# EUROPEAN PATENT SPECIFICATION

(11) **EP 3 737 933 B1**
(45) Date of publication and mention of the grant of the patent: **15.11.2023**
(21) Application number: 19704377.1
(22) Date of filing: 02.01.2019
(51) Int. Cl.: G01N 21/78, G01N 33/18, C12Q 1/04, G01N 21/03

(54) **WATER QUALITY TESTING**
WASSERQUALITÄTSPRÜFUNG
TEST DE LA QUALITÉ DE L'EAU

(30) Priority: 09.01.2018 GB 201800303
(43) Date of publication of application: 18.11.2020
(73) Proprietor: Molendotech Limited, Brixham, Devon TQ5 8BA (GB)
(72) Inventor: JACKSON, Simon, Brixham, Devon TQ5 8BA (GB); SATTAR, Anas, Brixham, Devon TQ5 8BA (GB); BRADLEY, Graham, Brixham, Devon TQ5 8BA (GB)
(74) Representative: Bazant-Hegemark, Florian
(86) International application number: PCT/GB2019/050002
(87) International publication number: WO 2019/138211

(56) References cited:
- CN-U- 202 189 009
- GB-A- 2 423 358
- JP-A- 2015 021 906
- US-A- 5 386 287

## Description

This invention relates to a method and apparatus for use in testing water quality and other fluids, and in particular to testing for faecal contamination thereof. The invention relates to a simple method and apparatus that allows testing for faecal contamination to be undertaken at the site from which a water or similar sample is taken, the output of the test being available rapidly, thereby providing a substantially real time indication of faecal contamination of the water or other sample. References herein to testing water quality are intended to refer to testing for the presence of faecal contamination of the water or other fluid, achieved by using faecal indicator bacteria as an indicator for the presence of such contamination.

There are a number of applications in which it is desired to be able to provide an indication of water quality, and in particular faecal contamination thereof. By way of example, where water based activities such as swimming, surfing, sailing and the like are to be undertaken, there is a desire to be able to obtain an indication of the quality of the water in which the activities are to be undertaken in order to provide an indication of whether or not the water quality is sufficiently good to allow the activities to be undertaken. Where water or other fluid is being used in food or drink production then there is a need to be able to ensure that the water quality or the like is high enough, and in particular that faecal contamination thereof is low enough, to allow the production steps being undertaken to be performed without negatively impacting upon the quality of the food or drink under production. One application in which it is desirable to ensure that water quality meets a predetermined specification is in the washing of fruit or vegetables, or other foodstuffs such as salad leaves or the like, prior to packaging for sale. Such washing may be undertaken in the region at which the products are grown, and if the water quality at that location is variable, then it is desirable to be able to undertake checks periodically in order to ensure, substantially in real time, that the water quality is sufficiently high to permit its use in the production process being undertaken. Without such tests being undertaken, the products may be inadvertently contaminated by the use of unacceptably poor quality water, rendering the products unsuitable for consumption or requiring additional cleaning steps to be taken. Another application in which water quality testing may be undertaken is in testing untreated or broken drinking water supplies from, for example, wells, rivers, pipelines, tanks and other sources. It has been shown that bacterial lipopolysaccharide (LPS) is a useful indicator of faecal contamination and can be used as such to screen water quality for the presence of faecal contamination.

US9664663 describes a water testing regime that is suitable for use in certain applications of this type, the method involving taking a sample of the water to be tested, diluting the sample, applying a reagent to the water sample, and heating the water sample for a predetermined period of time. The reagent is sensitive to a presence of bacterial LPS, changing colour in the event that the presence of LPS is detected, and the presence of LPS is used as a marker to provide an indication of the water quality by providing an indication of the level of faecal bacteria therein. The presence of the LPS in the presence of the reagent causes the water sample to change colour, and the colour of the water sample can be used to provide an indication of water quality at the test location.

US5386287, CN202189009U and JP2015021906 describe test methods and apparatuses.

It is an object of the invention to provide a method, making use of the testing regime described in US9664663, which is simple and convenient to use.

According to the present invention there is provided a water quality testing method as defined by appended Claim 1. As discussed above, the water quality testing method operates to detect the level of faecal contamination of the water, and thereby provides an indication of the presence of live colony forming units of bacteria in the water. It is not intended to detect for the presence of other contamination.

The water sample or the like may be diluted with water, if desired.

The reagent may be of tablet or powdered form, or could be of liquid form. The reagent may contain a preservative such as mannitol to increase the shelf life of the reagent.

The colour or darkness may be detected using a suitable detector.

Such a method is advantageous in that the reagent may be provided in a form that is convenient for use. The use of a tablet is advantageous in that the correct dose of reagent will be used. Similarly, where the reagent is pre-packaged in other single dose forms or within a single use reaction chamber, it can be ensured that the correct quantity of the reagent is present.

The reagent is preferably lyophilised and may be processed to take tablet form or may be pre-packaged within a single use reaction chamber.

Analysis of the colour or darkness of the reaction solution may be undertaken using a spectrophotometer.

A further reagent is present in the reaction chamber to stabilise the reaction solution and interrupt the reaction.

If desired, another further reagent may be added to present in the reaction chamber, for example to enhance or simplify reading of the change in colour or darkness.

The incubation step is preferably undertaken at a temperature in the range of 15-45ºC, preferably at 37ºC, and preferably is undertaken for a period of time in the region of 5 to 60 minutes, preferably approximately 30 minutes.

The invention further relates to a test apparatus as defined by appended Claim 6.

The reagent may be in tablet form, placed within the reaction chamber. However, other forms including powders and liquids may be used. Alternatively, the reagent may be pre-packaged within a single use reaction chamber.

Such an apparatus is convenient to use as the operator need only perform a minimal number of steps. The risk of operator error resulting in inaccurate results is thus reduced.

The invention further relates to a LAL or TAL reagent supplied in tablet form, or supplied within single use reaction chambers. The reagent is preferably supplied in lyophilised form.

The invention will further be described, by way of example, with reference to the accompanying drawings, in which:
Figure 1 is a diagram illustrating a method in accordance with an embodiment of the invention;
Figure 2 is a diagram illustrating an apparatus in accordance with an embodiment of the invention; and
Figure 3 is a diagram illustrating a method that may be used in the production of the reagent in tablet form that may be used in the method and apparatus of the invention.

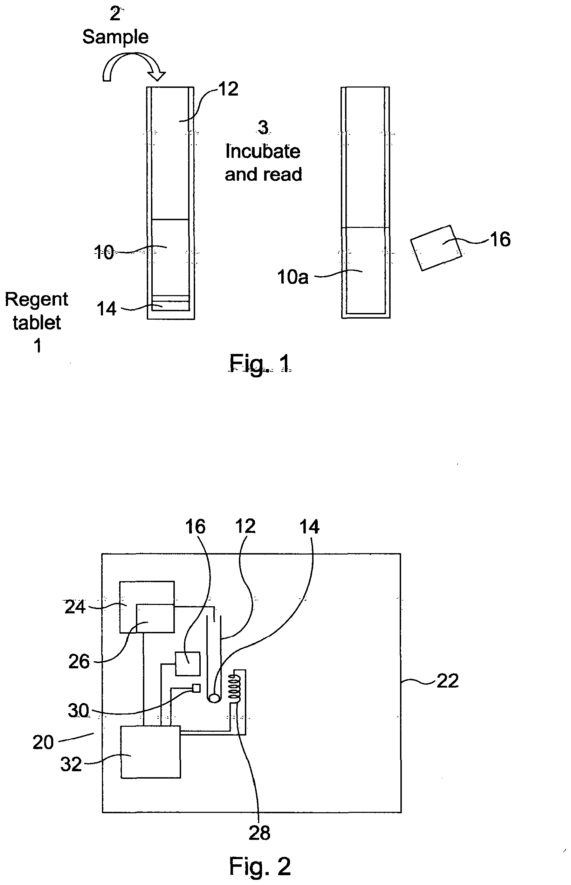

Referring firstly to Figure 1, a method in accordance with an embodiment of the invention consists of the steps of adding a sample 10 of water from a water source or another fluid to be tested to a reaction chamber 12. In the arrangement shown, the reaction chamber takes the form of a test tube. It will be appreciated, however, that the reaction chamber 12 may take a range of other forms. Also located within the reaction chamber 12 is a tablet 14 of a reagent. Whilst a tablet 14 is shown, it will be appreciated that the reagent may be in other forms, for example it may be in powdered or liquid form.

The water sample 10 could take the form of water taken from a body of water such as a lake, river, a swimming pool or the sea in which an activity is to be undertaken.

Alternatively, it could comprise tap water from a piped water supply, water extracted from a well or borehole, or water from a water tank, storage reservoir or the like. In addition, the sample may be of other fluids from, for example, the food and beverage industry. It will be appreciated that these are merely examples and that the sample may be taken from a range of other sources, depending upon the application in which the invention is to be employed. If desired, the sample may be diluted with water prior to testing.

The reagent comprises a Limulus Polyphemus derived enzyme (LAL) or a Tachypleus tridentatus derived enzyme (TAL) that is preferably lyophilised and formed into a single dose-sized tablet. However, as mentioned above, it may take other forms. If desired, it may contain a preservative to increase its shelf life. For example, mannitol may be used.

The reaction chamber 12 containing the sample 10 and the tablet 14 is incubated at a temperature in the range of 15 to 45ºC for a period of 5 to 60 minutes. Preferably, incubation is undertaken at a temperature of 37ºC for approximately 30 minutes. After incubation, the reagent within the tablet 14 will have reacted with the sample 10, forming a coloured reaction solution 10a. The colour or darkness of the reaction solution 10a provides an indication of the level of LPS present within the sample. The level of LPS can be used as a marker to provide an indication of the level of faecal bacteria within the sample, and hence to provide an indication of the sample water quality.

In the arrangement of Figure 1, the colour or darkness of the reaction solution 10a, after completion of the incubation step, is identified using a handheld spectrophotometer device 16. By way of example, the spectrophotometer device 16 may be arranged to irradiate the reaction solution 10a with light of a predetermined wavelength, and to quantify the reflectance or absorption of the light by the solution 10a, providing an indication of the colour or darkness of the reaction solution 10a. Comparison of the level of reflectance or absorption with reference information can be used to provide an output indicative of the water quality of the sample 10. In particular, it provides an indication of the presence of faecal contamination in the water sample.

The method described hereinbefore is advantageous in that it is simple to use. It is of single step form, simply requiring the addition of the sample 10 to a reaction chamber containing or preloaded with a dose of the reagent. The provision of the reagent in the form of a tablet 14 ensures that the correct quantity of the reagent is present, and the use of the spectrophotometer allows an accurate assessment of the colour or darkness of the sample 10. The method thus enables water quality to be tested to an increased level of accuracy.

Depending upon the nature of the sample, there may be a need to dilute the sample before exposing it to the reagent. This may be required where the sample is of, for example, saline form.

To further enhance the accuracy with which water quality can be measured, the method may include a step of adding one or more further reagents to the sample. By way of example, the further reagents may include a stop reagent which operates to interrupt the reaction between the sample 10 and the tablet 14. Alternatively, or additionally, the further reagents may serve to enhance the change in colour or darkness, to allow better discrimination thereof and hence allow a more accurate indication of the water quality.

Whilst in the description hereinbefore, the reagent is in the form of a tablet 14, it will be appreciated that the invention is also applicable to arrangements in which the reagent is in other single dose forms, such as in suitable single dose powder or liquid packages, or is pre-packaged, sealed within a single use reaction chamber. As with the use of a tablet, this is advantageous in that it aids in ensuring that the correct quantity of the reagent is used in the test, enhancing the accuracy with which water quality can be measured. It has the further benefit that errors arising from the use of a contaminated reaction chamber can be avoided. Where pre-packaged in this manner, the reagent could take the form of, for example, a powder or liquid.

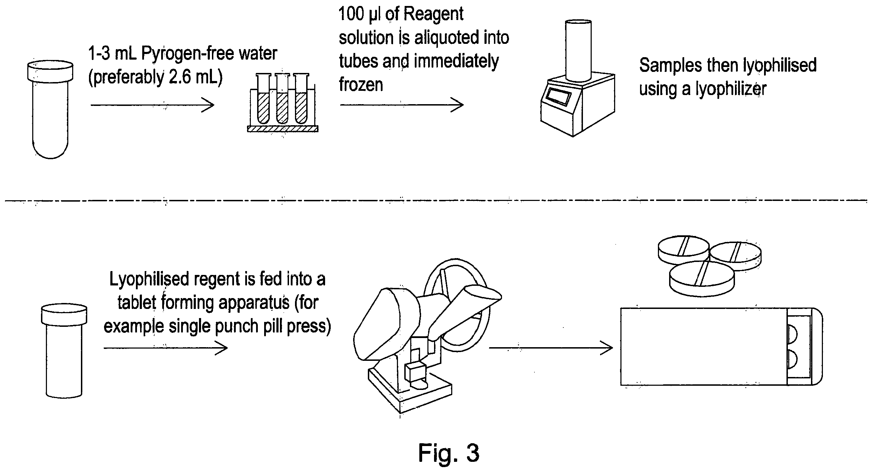

The reagent may be obtained, as shown in Figure 3, by solubilising a lyophilised LAL or TAL reagent with 1 to 3 ml of pyrogen-free water. The amount of water may be varied to take into account variations in the supplied LAL or TAL regent. Typically, 2.6 ml will be used. The solubilised reagent is then aliquoted, for example into 100µl doses. One or more preservatives such as mannitol may be added to the reagent to prolong the shelf life of the lyophilized powder or tablet form. The reagent is then frozen at, for example -20ºC or lower, for at least 5 hours and lyophilised for at least 24 hours to form a powder. The powder can be formed into individual dose tablets 14 or may be packaged and sealed within individual dose, single use test tubes or the like.

The method described hereinbefore lends itself to use in an automated test apparatus, for example of the form shown in Figure 2. As shown in Figure 2, the apparatus 20 comprises a housing 22 within which a sample reservoir 24 is located. A metering device 26, for example in the form of a stepper motor driven positive displacement pump, is operable to supply a predetermined, controlled quantity of a water sample 10 from the reservoir 24 to a reaction chamber 12 in the form of a removable test tube containing a tablet 14 of the reagent as described hereinbefore, or a single use test tube pre-packaged with a single dose of the reagent as described hereinbefore. The apparatus 20 further comprises a heater 28 operable to heat the sample, and a temperature probe 30 operable to measure a temperature of the sample. The metering device 26 and the heater 28 are controllable by a control unit 32 which receives temperature information from the probe 30. The control unit 32 incorporates a timer.

The apparatus 20 further comprises a spectrophotometer device 16 operable under the control of the control unit 32 to determine the colour or darkness of the reaction solution resulting from the use of the apparatus.

In use, the reaction chamber 12 and tablet 14 are positioned within the apparatus 20, and a quantity of water, the quality of which is to be tested is placed within the reservoir 24. The control unit 32 is instructed to undertake testing of the water, and this is undertaken by controlling the metering device 26 to transfer a metered quantity of water to the reaction chamber 12 containing the tablet 14, controlling the heater 28, using the output of the probe 30, to incubate the sample at the desired temperature for the desired period of time to form a coloured reaction solution, and then using the spectrophotometer to ascertain, from the colour or darkness of the reaction solution, the level of faecal bacteria present within the sample and hence the quality of the water from which the sample is taken. The apparatus 20 may also be connected for example via a USB port or via a Bluetooth device to allow data to be exported for analysis and recording at remote sites.

It will be appreciated that by automating many of the test steps, the test may be undertaken by a lay person without specialist training, in a non-laboratory environment, whilst still ensuring that the test results are achieved to an acceptable level of accuracy.

Although one specific embodiment of the invention is described hereinbefore, it will be appreciated that a number of modifications and alterations may be made thereto without departing from the scope of the invention as defined by the appended claims.

## Claims

1. A water quality testing method comprising the steps of placing a water sample (10) within a reaction chamber (12) containing a single dose of a reagent, or placing the water sample within a single use reaction chamber pre-packaged with a single dose of the reagent, the reaction chamber (12) or single use reaction chamber further containing a further reagent to stabilise the sample (10), incubating the sample (10) at a predetermined temperature for a predetermined period of time to form a coloured reaction solution (10a), and using the resulting colour or darkness of the reaction solution (10a) to provide an indication of the water quality, wherein the reagent is sensitive to a presence of bacterial lipopolysaccharide (LPS), changing colour or darkness in the event that the presence of LPS is detected, the presence of LPS being used as a marker to provide an indication of the water quality by providing an indication of the level of faecal bacteria in the sample, the reagent comprising an enzyme derived from Limulus Polyphemus (LAL) or Tachypleus Tridentatus (TAL), the further reagent interrupting the reaction between the sample and the reagent.

2. A method according to claim 1, wherein the reagent is lyophilised or prepared in tablet form.

3. A method according to claim 1 or 2, wherein analysis of the colour or darkness of the reaction solution (10a) is undertaken using a spectrophotometer.

4. A method according to any of the preceding claims, wherein another further reagent is added to or present in the reaction chamber (12) to enhance or simplify reading of the change in colour or darkness of the sample.

5. A method according to any of the preceding claims, wherein the incubation step is undertaken at a temperature in the range of 15-45°C, for a period of time in the region of 5 to 60 minutes.

6. A test apparatus adapted for performing the method of any of the preceding claims and comprising a reaction chamber (12), a reagent located within the reaction chamber (12), the reaction chamber (12) further containing a further reagent, metering means for adding a metered quantity of a sample (10) to the reaction chamber (12), a heater for heating the reaction chamber to allow incubation of the sample (10) located therein at a predetermined temperature for a predetermined period of time, and a spectrophotometer operable to allow analysis of the colour of the sample, after incubation, to ascertain an indication of the water quality of the sample, wherein the reagent is sensitive to a presence of bacterial lipopolysaccharide (LPS), changing colour or darkness in the event that the presence of LPS is detected, the presence of LPS being used as a marker to provide an indication of the water quality by providing an indication of the level of faecal bacteria in the sample, the reagent comprising an enzyme derived from Limulus Polyphemus (LAL) or Tachypleus Tridentatus (TAL), the further reagent serving to stabilise the sample and interrupt the reaction between the sample and the reagent.

7. An apparatus according to claim 6, wherein the reagent is in tablet form, placed within the reaction chamber (12).

8. An apparatus according to claim 6, wherein the reagent is pre-packaged within a single use reaction chamber (12).

## Patentansprüche

1. Wasserqualitätprüfverfahren, umfassend die Schritte eines Einbringens einer Wasserprobe (10) in eine Reaktionskammer (12), die eine Einzeldosis eines Reagens enthält, oder eines Einbringens der Wasserprobe in eine Einweg-Reaktionskammer, die mit einer Einzeldosis des Reagens vorverpackt ist, wobei die Reaktionskammer (12) oder die Einweg-Reaktionskammer ferner ein weiteres Reagens enthält, um die Probe (10) zu stabilisieren, die Probe (10) bei einer vorbestimmten Temperatur über eine vorbestimmte Zeitdauer zu inkubieren, um eine gefärbte Reaktionslösung (10a) zu bilden, und die resultierende Farbe oder Dunkelheit der Reaktionslösung (10a) zu verwenden, um eine Angabe der Wasserqualität bereitzustellen, wobei das Reagenz empfindlich auf das Vorhandensein von bakteriellem Lipopolysaccharid (LPS) reagiert und Farbe oder Dunkelheit ändert, wenn das Vorhandensein von LPS erfasst wird, wobei das Vorhandensein von LPS als Marker verwendet wird, um eine Angabe der Wasserqualität bereitzustellen, indem es eine Angabe des Gehalts an Fäkalbakterien in der Probe bereitstellt, das Reagenz umfassend ein Enzym, das von Limulus Polyphemus (LAL) oder Tachypleus Tridentatus (TAL) stammt, wobei das weitere Reagenz die Reaktion zwischen der Probe und dem Reagenz unterbricht.

2. Verfahren nach Anspruch 1, wobei das Reagenz lyophilisiert oder in Tablettenform hergestellt wird.

3. Verfahren nach Anspruch 1 oder 2, wobei eine Analyse der Farbe oder Dunkelheit der Reaktionslösung (10a) unter Verwendung eines Spektralphotometers vorgenommen wird.

4. Verfahren nach einem der vorherigen Ansprüche, wobei zu der Reaktionskammer (12) ein weiteres Reagenz hinzugefügt wird oder darin vorhanden ist, um ein Ablesen der Farb- oder Dunkelheitsänderung der Probe zu verbessern oder zu vereinfachen.

5. Verfahren nach einem der vorherigen Ansprüche, wobei der Inkubationsschritt bei einer Temperatur in dem Bereich von15-45 °C über eine Zeitdauer in dem Bereich von 5 bis 60 Minuten vorgenommen wird.

6. Prüfvorrichtung, die zum Ausführen des Verfahrens nach einem der vorherigen Ansprüche angepasst ist, umfassend eine Reaktionskammer (12), ein in der Reaktionskammer (12) befindliches Reagenz, wobei die Reaktionskammer (12) ferner ein weiteres Reagenz enthält, Dosiereinrichtungen zum Hinzufügen einer dosierten Menge einer Probe (10) in die Reaktionskammer (12), eine Heizung zum Erwärmen der Reaktionskammer, um eine Inkubation der darin befindlichen Probe (10) bei einer vorbestimmten Temperatur über eine vorbestimmte Zeitdauer zu ermöglichen, und ein Spektralphotometer, der betrieben werden kann, um eine Analyse der Farbe der Probe nach Inkubation zu ermöglichen, um eine Angabe der Wasserqualität der Probe zu ermitteln, wobei das Reagenz empfindlich auf das Vorhandensein von bakteriellem Lipopolysaccharid (LPS) reagiert und Farbe oder Dunkelheit ändert, wenn das Vorhandensein von LPS erfasst wird, wobei das Vorhandensein von LPS als Marker verwendet wird, um eine Angabe der Wasserqualität bereitzustellen, indem es eine Angabe des Gehalts an Fäkalbakterien in der Probe bereitstellt, das Reagenz umfassend ein Enzym, das von Limulus Polyphemus (LAL) oder Tachypleus Tridentatus (TAL) stammt, wobei das weitere Reagenz zum Stabilisieren der Probe und Unterbrechen der Reaktion zwischen der Probe und dem Reagenz dient.

7. Vorrichtung nach Anspruch 6, wobei das Reagenz in Tablettenform ist, in die Reaktionskammer (12) eingebracht wird.

8. Vorrichtung nach Anspruch 6, wobei das Reagenz in einer Einweg-Reaktionskammer (12) vorverpackt ist.

## Revendications

1. Procédé de test de la qualité de l'eau comprenant les étapes de placement d'un échantillon d'eau (10) à l'intérieur d'une chambre de réaction (12) contenant une dose unique d'un réactif, ou le placement de l'échantillon d'eau à l'intérieur d'une chambre de réaction à usage unique préemballée avec une dose unique du réactif, la chambre de réaction (12) ou la chambre de réaction à usage unique contenant en outre un réactif supplémentaire pour stabiliser l'échantillon (10), incubation de l'échantillon (10) à une température prédéfinie pendant une période de temps prédéfinie pour former une solution réactionnelle colorée (10a), et utilisation de la couleur ou du noircissement résultant de la solution réactionnelle (10a) pour fournir une indication de la qualité de l'eau, ledit réactif étant sensible à la présence d'un lipopolysaccharide bactérien (LPS), changeant de la couleur ou de la noirceur dans le cas où le la présence de LPS est détectée, la présence de LPS étant utilisée en tant que marqueur pour fournir une indication de la qualité de l'eau en fournissant une indication du taux de bactéries fécales dans l'échantillon, le réactif comprenant une enzyme dérivée de Limulus Polyphemus (LAL) ou Tachypleus Tridentatus (TAL), le réactif supplémentaire interrompant la réaction entre l'échantillon et le réactif.

2. Procédé selon la revendication 1, ledit réactif étant lyophilisé ou préparé sous forme de comprimé.

3. Procédé selon la revendication 1 ou 2, ladite analyse de la couleur ou de la noirceur de la solution réactionnelle (10a) étant entreprise à l'aide d'un spectrophotomètre.

4. Procédé selon l'une quelconque des revendications précédentes, un autre réactif supplémentaire étant ajouté ou présent dans la chambre de réaction (12) pour améliorer ou simplifier la lecture du changement de couleur ou de noirceur de l'échantillon.

5. Procédé selon l'une quelconque des revendications précédentes, ladite étape d'incubation étant entreprise à une température comprise dans la plage de 15-45°C, pendant une période de temps comprise dans la zone de 5 à 60 minutes.

6. Appareil de test adapté pour réaliser le procédé selon l'une quelconque des revendications précédentes et comprenant une chambre de réaction (12), un réactif situé à l'intérieur de la chambre de réaction (12), la chambre de réaction (12) contenant en outre un réactif supplémentaire, des moyens de dosage pour ajouter une quantité dosée d'un échantillon (10) à la chambre de réaction (12), un dispositif de chauffage destiné à chauffer la chambre de réaction afin de permettre l'incubation de l'échantillon (10) situé à l'intérieur de celle-ci à une température prédéfinie pendant une période de temps prédéfinie, et un spectrophotomètre utilisable pour permettre l'analyse de la couleur de l'échantillon, après incubation, pour déterminer une indication de la qualité de l'eau de l'échantillon, ledit réactif étant sensible à une présence de lipopolysaccharide bactérien (LPS), changeant de couleur ou de noirceur dans le cas où la présence de LPS est détectée, la présence de LPS étant utilisée en tant que marqueur pour fournir une indication de la qualité de l'eau en fournissant une indication du taux de bactéries fécales dans l'échantillon, le réactif comprenant une enzyme dérivée de Limulus Polyphemus (LAL) ou Tachypleus Tridentatus (TAL), le réactif supplémentaire servant à stabiliser l'échantillon et à interrompre la réaction entre l'échantillon et le réactif.

7. Appareil selon la revendication 6, ledit réactif étant sous forme de comprimé, placé à l'intérieur de la chambre de réaction (12).

8. Appareil selon la revendication 6, ledit réactif étant préemballé à l'intérieur d'une chambre de réaction à usage unique (12).
